# EUROPÄISCHE PATENTSCHRIFT

(11) **EP 2 478 921 B1**
(45) Veröffentlichungstag und Bekanntmachung des Hinweises auf die Patenterteilung: **02.09.2015**
(21) Anmeldenummer: 12000050.0
(22) Anmeldetag: 05.01.2012
(51) Int. Cl.: A61L 2/26, A61B 19/02

(54) **Sterilisierbehälter mit Filtereinheit**
Sterilisation container with filter unit
Récipient de stérilisation doté d'une unité de filtre

(30) Priorität: 21.01.2011 DE 202011001772 U
(43) Veröffentlichungstag der Anmeldung: 25.07.2012
(73) Patentinhaber: Innovations Medical GmbH, 78532 Tuttlingen (DE)
(72) Erfinder: Kreidler, Winfried, 78532 Tuttlingen (DE)
(74) Vertreter: Binner, Bernhard

(56) Entgegenhaltungen:
- EP-A1- 1 563 854
- EP-A1- 2 356 952
- WO-A1-03/041748
- WO-A1-03/041749
- DE-U1-202010 009 925

## Beschreibung

Die Erfindung betrifft einen Sterilisierbehälter bestehend aus einem kastenartigen Unterteil und einem abnehmbaren Behälterdeckel, welche einen geschlossenen, luftdichten Sterilraum bilden, wobei in einem Wandbereich des Sterilisierbehälter Belüftungsöffnungen vorgesehen sind, durch welche im geschlossenen Zustand ein Luftaustausch zwischen dem Sterilraum und der Umgebung stattfindet, und wobei der Wandbereich im Bereich der Belüftungsöffnungen zusammen mit einer Deckelplatte Bestandteil einer Sterilbarriere ist, welche eine Sterilisation der durch die Belüftungsöffnungen in den Sterilraum gelangenden Luft bewirkt, wobei die Deckelplatte durch wenigstens eine federnde Schnapp- oder Rastverbindung lösbar mit dem Wandbereich in Eingriff steht.

Sterilisierbehälter mit einem Untergehäuse sowie einem dicht aufsetzbaren Behälterdeckel sind schon seit langem bekannt und werden beispielsweise zur Sterilisation von medizinischen Geräten eingesetzt. Um bei geschlossenem Sterilisierbehälter eine Be- und Entlüftung zu gewährleisten, sind solche Sterilisierbehälter in wenigstens einem ihrer Wandbereiche mit Gasaustauschöffnungen versehen, welche beispielsweise im Betrieb innenseitig mittels eines Filterblattes abgedeckt sein können. Da mit zunehmender Betriebsdauer ein solches Filterblatt auszutauschen ist, wird ein solches Filterblatt mittels einer Andruckscheibe auswechselbar innenseitig am Wandbereich gehalten. Dementsprechend ist die Andruckscheibe innenseitig am Wandbereich angeordnet und steht mit einem die Gasaustauschöffnungen ringförmig umgebenden Rahmenelement lösbar in Eingriff. Dabei kann diese lösbare Verbindung zwischen der Andruckscheibe und dem Rahmenelement durch eine Renkverbindung hergestellt sein, welche aus wechselseitig miteinander in Eingriff bringbaren Renkverschlusselementen bzw. Renkverbindungselementen an der Andruckscheibe einerseits und am Rahmenelement andererseits gebildet werden. Zur Abdichtung der Andruckscheibe gegenüber dem entsprechenden Wandbereich des Sterilisierbehälters bzw. gegenüber dem Rahmenelement ist wenigstens ein Dichtungsring vorgesehen. Dieser Dichtungsring wird wandseitig in einer umlaufenden Ringnut der Andruckscheibe oder des Deckelbodens aufgenommen. Um eine gute Dichtwirkung zu erreichen, drückt dieser Dichtungsring im montierten Zustand gegen das Filterblatt, welches vorzugsweise - relativ passgenau - in das ringförmige Rahmenelement eingesetzt ist. Damit bildet die Andruckscheibe zusammen mit dem Filterblatt und dem Rahmenelement eine Art Filtereinheit.

Aufgrund der vorgesehenen Renkverbindung zwischen der Andruckscheibe und dem Rahmenelement kann die Andruckscheibe nur durch eine drehende Relativbewegung aus dem Rahmenelement gelöst und wieder in dieses eingesetzt werden. Da der Dichtungsring der Andruckscheibe dichtend gegen das Filterblatt drückt und die Andruckscheibe dementsprechend unter axialer Vorspannung gegen das Filterblatt zu verspannen ist, führt diese relative Drehbewegung insbesondere bei der Montage der Andruckscheibe zu einer Verschiebung des Filterblattes und/oder zu dessen Beschädigung.

Beim Gegenstand der WO 03/041749 A1 sind ebenfalls Renkverbindungen vorgesehen. Das Filterblatt wird bei dieser bekannten Konstruktion zwischen zwei mit Durchbrüchen versehenen Klemmscheiben gehalten, welche fest miteinander verbunden sind. Damit bilden diese Klemmscheiben zusammen mit dem Filterblatt eine komplette Filtereinheit, welche in einem entsprechenden Wandbereich oder Deckels eines Sterilbehälters eines Sterilbehälters über einen Filterhalter einsetzbar ist. Zur Fixierung dieser Filtereinheit im Filterhalter ist eine Abdeckung vorgesehen, welche über eine Renkverbindung oder eine Art Bajonettverbindung auf den Filterhalter aufsetzbar und über eine Drehbewegung feststehend mit diesem verbindbar ist. Da das Filterblatt zwischen den beiden Klemmscheiben aufgenommen ist, findet keinerlei Kontakt zwischen der Abdeckung und dem Filterblatt während dieser Drehbewegung statt. Eine Beschädigung des Filterblattes ist hier folglich nicht zu befürchten. Allerdings ist ein Austausch des Filterblattes als Einzelelement nicht möglich, da die beiden Klemmschieben miteinander verklebt oder verschweißt sind, so dass stets die gesamte Filtereinheit ausgetauscht werden muss.

Insoweit wäre es äußerst vorteilhaft, diese Renkverbindung durch eine andere Verbindungsart zu ersetzen, um eine derartige Zerstörung bzw. Verschiebung des Filterblattes zu vermeiden.

Hierzu ist aus der DE 20 2010 001 382 U1 ein Sterilisierbehälter bekannt, welcher ebenfalls aus einem kastenartigen Unterteil und einem Behälterdeckel besteht, der dicht auf das Unterteil aufsetzbar ist. Bei diesem Sterilisierbehälter ist allerdings als Filtereinheit kein Filterblatt vorgesehen, sondern eine sog. Pasteursche Schleife. Eine solche Pasteursche Schleife besteht in der Regel aus zwei Platten, nämlich einer Sockelplatte und einer Deckelplatte, die jeweils konzentrische Ringrippen aufweisen. Diese Ringrippen der Sockelplatte und der Deckelplatte greifen mit radialem Spiel axial derart ineinander, dass sowohl axiale als auch radiale Ringspalten gebildet werden. Des Weiteren ist im Bereich dieser Pasteurschen Schleife ein Wandabschnitt des Behälterdeckels mit Belüftungsöffnungen versehen, durch welche im Betrieb eine Be- und Entlüftung des geschlossenen Sterilisierbehälters erfolgt. Durch die Pasteursche Schleife wird die durch die Belüftungsöffnungen eintretende und diese Ringspalte abwechselnd radial und axial durchströmende und schließlich in den Sterilisierraum gelangende Luft bzw. aus diesem entweichende Luft sterilisiert.

Beim Gegenstand der DE 20 2010 001 382 U1 ist die Sockelplatte, welche mittels Schraubverbindungen oder dgl. innenseitig am Wandelement des Behälterdeckels montiert ist, mit ihren Ringrippen als komplettes separates Bauteil ausgebildet. Die Deckelplatte ist in einer Ausführungsvariante mittels mehrerer gleichmäßig am Umfang verteilter, federbelasteter Rastkugeln rastend mit der Sockelplatte in Eingriff bringbar. Diese Rastkugeln sind dabei in einem axial vorstehenden, umlaufenden Haltering angeordnet, welcher die Deckelplatt umgibt. Durch die separate Ausbildung der Sockelplatte sind insbesondere die Querbohrungen zur Aufnahme der Rastkugeln sowie der diese belastenden Axialdruckfedern in äußerst einfacher Weise anbringbar. Allerdings hat sich gezeigt, dass es hierzu zwingend erforderlich ist, diese Sockelplatte als separates Bauteil auszubilden und die entsprechenden Querbohrungen im Randsteg anzubringen und die Rastkugeln vor der Montage einzusetzen. Erst danach kann die Sockelplatte innenseitig auf den Wandabschnitt des Behälterdeckels mit seinen Belüftungsöffnungen aufgesetzt werden. Diese spezielle Konstruktion der Rastverbindung wäre auch zur Fixierung eines Filterblattes einsetzbar. Jedoch ist hier stets, um die Querbohrungen im Randsteg anfertigen zu können, eine separate Ausbildung der Sockelplatte notwendig.

Demgemäß liegt der Erfindung die Aufgabe zugrunde, eine Rastverbindung zur Montage der Deckelplatte derart auszugestalten, dass die Sockelplatte einstückiger, integraler Bestandteil des entsprechenden Wandabschnittes mit seinen Belüftungsöffnungen ist.

Die Aufgabe wird erfindungsgemäß zusammen mit den Merkmalen des Oberbegriffes des Anspruches 1 dadurch gelöst, dass die Deckelplatte als runde Scheibe mit einer umlaufenden Ringwand ausgebildet ist, die außenseitig eine umlaufende Rastnut aufweist, in welche mehrere im Umfangsbereich der Deckelplatte angeordnete Rastelemente lösbar eingreifen und, dass die Rastelemente als jeweils separate Baueinheit in rechtwinklig zur Deckelplatte verlaufende Aufnahmebohrungen von axial über den Wandbereich vorstehenden Ringssegmenten feststehend angeordnet sind.

Durch die erfindungsgemäße Ausgestaltung kann eine zusätzliche Sockelplatte zum Anordnen bzw. Anbringen der äußeren Rastelemente entfallen. So sind am Behälterboden bzw. am Wandelement des Sterilisierbehälters mehrere auf einer Kreisbahn angeordnete domartige Vorsprünge oder Ringsegmente vorgesehen, welche mit axialen bzw. quer zum Wandelement verlaufenden Aufnahmebohrungen versehen sind. In diese Aufnahmebohrungen ist ein aus mehreren Teilen bestehendes Rastelement feststehend einsetzbar. Durch entsprechende Wahl der Anordnung dieser Ringsegmente ist somit eine Deckelplatte präzise positioniert und ohne jegliche Drehbewegung innenseitig im entsprechenden Wandbereich des Sterilisierbehälters einrastbar.

Erfindungsgemäß können die Ringsegmente mit ihren Rastelementen auch Bestandteil der Deckelplatte sein. In einem solchen Fall befindet sich die Ringnut in einem umlaufenden, die Deckelplatte umschließenden Ringsteg des entsprechenden Wandbereiches.

Weitere vorteilhafte Ausgestaltungen der Erfindung sind den weiteren Unteransprüchen zu entnehmen.

Die Merkmalskombinationen der Ansprüche 2 bis 4 betreffen insbesondere vorteilhafte Weiterbildungen des Rastelementes.

So kann gemäß Anspruch 2 vorgesehen sein, dass die Rastelemente jeweils aus einem etwa zylindrischen Gehäuseblock bestehen, welcher mit einer quer verlaufenden Radialbohrung versehen ist, in welcher eine radial aus dem Gehäuseblock teilweise vorstehende und im montierten Zustand in die Rastnut eingreifende Rastkugel angeordnete ist und, dass die Rastkugel durch eine in der Radialbohrung angeordnete Axialdruckfeder in ihrer Eingriffsposition federelastisch nachgiebig gehalten ist.

Gemäß Anspruch 3 kann vorgesehen sein, dass die Radialbohrung im Bereich der teilweise radial vorstehenden Rastkugel einen nach innen gerichteten Anschlagsteg aufweist, durch welchen der maximale radiale Vorstand der Rastkugel bestimmt ist.

Weiter kann gemäß Anspruch 4 die Radialbohrung des Gehäuseblocks in ihrem der Rastkugel gegenüber liegenden Endbereich mit einer Verschlussplatte versehen sein, welche in einer Ausfräsung des Gehäuseblocks feststehend aufgenommen ist und an welcher sich die Axialdruckfeder axial abstützt.

In einer Variante der Erfindung kann die Filtereinheit des Sterilisierbehälters gemäß Anspruch 5 zwischen der Deckelplatte und dem Wandbereich des Behälterdeckels ein die Belüftungsöffnungen innenseitig abdeckendes Filterblatt aufweisen. Bei dieser Ausgestaltung bewirkt die Rastverbindung gemäß Anspruch 5 zwischen der Deckelplatte und dem Wandbereich eine Klemmkraft, mit welcher das Filterblatt gegen den Wandbereich des Behälterdeckels gedrückt wird. zwischen der Deckelplatte und dem Wandbereich des Behälterdeckels ein die Belüftungsöffnungen innenseitig abdeckendes Filterblatt vorgesehen sein.

Insbesondere kann gemäß Anspruch 6 für eine derartige Ausgestaltung die Deckelplatte ein radial vorstehendes Griffteil aufweisen, welches im montierten Zustand zwischen zwei benachbarten Ringsegmenten der Deckelplatte angeordnet ist, wobei das Griffteil zur inneren Oberfläche der Deckelwand des Behälterdeckels einen Abstand aufweist. Durch dieses Griffteil ist die zwischen der Deckelplatte und den Rastelementen bestehende Rastverbindung in einfacher Weise durch manuelles Anheben des Griffteils und somit der gesamten Deckelplatte aufhebbar. Durch den Abstand des Griffteils zur inneren Oberfläche der Deckelwand kann das Griffteil sicher manuelle gegriffen werden. Durch das vorgesehene Griffteil ist auch eine Relativdrehung der Deckelplatte relativ zum eingelegten Filterblatt zur Aufhebung der Rastverbindung nicht notwendig, so dass das Filterblatt insbesondere für deren evtl. Wiederverwendung keinen Schaden nehmen kann.

Um das Griffteil sicher mit den Fingern "hintergreifen" zu können, kann zusätzlich zu dieser Ausgestaltung nach Anspruch 7 das Griffteil auf seiner zur Oberfläche des Behälterdeckels hin liegenden "Unterseite" eine Abschrägung aufweisen, in dessen radial äußerem Endbereich der Abstand zur Oberfläche des Behälterbodens größer ist als in dessen radial innerem Bereich.

Gemäß Anspruch 8 kann die Filtereinheit und damit die Sterilbarriere alternativ zur Ausgestaltung nach Anspruch 5 aus einer Pasteurschen Schleife bestehen. Diese Pasteurschen Schleife wird aus in radialer Richtung wechselseitig axial ineinander greifenden Ringstegen des Wandbereichs einerseits und der Deckelplatte andererseits gebildet. Da hier kein Filterblatt vorgesehen ist, kann die Deckelplatte relativ zum Wandbereich gedreht werden, da keine Zerstörung eines solchen Filterblattes zu befürchten ist.

Dementsprechend ist hier eine alternative Ausgestaltung zum Lösen der Rastverbindung vorgesehen. So kann gemäß Anspruch 8 die Rastnut durch mehrere Aussparungen unterbrochen sein, deren Anzahl und Anordnung der Anzahl und der Anordnung der Rastelemente entsprechen, wobei die Aussparungen ausgehend von der Rastnut zum Wandbereich des Behälterdeckels hin offen sind.

Die Rastverbindung kann auch bei dieser Ausgestaltung durch Abheben der Deckelplatte aufgehoben werden. Für eine einfachere Aufhebung der Rastverbindung kann gemäß Anspruch 9 vorgesehen sein, dass die Deckelplatte relativ zum Wandbereich des Behälterdeckels drehbar ist und, dass die Aussparungen durch entsprechenden Einstellung der relativen Winkelstellung der Deckelplatte zum Wandbereich mit den im Umfangsbereich der Deckelplatte angeordneten Rastelementen in Überdeckung und mit der Rastnut außer Eingriff bringbar sind.

Zur Erleichterung der Einstellung dieser relativen Winkelstellung der Deckelplatte zum Behälterdeckel können gemäß Anspruch 10 im Umgebungsbereich der Deckelplatte auf dem Behälterdeckel und im Randbereich der Deckelplatte Markierungen vorgesehen sein, welche radial fluchtend zu einander angeordnete sind, sofern sich die Rastelemente im Umfangsbereich der jeweils zugeordneten Aussparung befinden.

Zur positionsgenauen Montage der Deckelplatte kann der Wandbereich des Sterilisierbehälters gemäß Anspruch 11 einen zentralen, zur Deckelplatte hin vorstehenden Lagerzapfen aufweisen, auf welchem die Deckelplatte im montierten Zustand mit einer Lagerbohrung konzentrisch zu den am Unfang angeordneten Ringsegmenten im Wandbereich aufgenommen ist.

Ist eine Ausführung der Deckelplatte ohne radial vorstehendes Griffteil vorgesehen, so kann der Abstand benachbarter Rastelemente gemäß Anspruch 12 jeweils derart gewählt sein, dass dazwischen ein manuelles Ergreifen der Deckelplatte an deren Umfang ermöglicht wird. Damit ist auch für eine derartige Ausgestaltung ein "Abziehen" oder "Abheben" der Deckelplatte in einfacher Weise möglich.

Anhand der Zeichnung wird nachfolgend beispielhaft die Erfindung anhand zweier Ausführungsvarianten näher erläutert. Es zeigt:
- Fig. 1: eine Innenansicht eines Behälterdeckels eines nicht weiter dargestellten Sterilisierbehälters, bei welchem in einem Wandbereich mehrere Belüftungsöffnungen vorgesehen sind und die Filtereinheit ein Filterblatt aufweist;
- Fig. 2: einen Teilschnitt II-II des Behälterdeckels aus Fig. 1;
- Fig. 3: eine perspektivische Explosionsdarstellung einer möglichen Ausführungsvariante eines Rastelementes;
- Fig. 4: eine perspektivische Darstellung eines Ringsegmentes, welches innenseitig über den Wandabschnitt des Behälterdeckels axial vorsteht und zur Aufnahme des Rastelementes aus Fig. 3 dient;
- Fig. 5: einen vergrößerten Ausschnitt V eines Ringsegmentes aus Fig. 1 mit einem eingesetzten Rastelement aus Fig. 3;
- Fig. 6: eine Schnittdarstellung VI-VI des Ringsegmentes aus Fig. 5 mit eingesetztem Rastelement;
- Fig. 7: eine Schnittdarstellung VII-VII aus Fig. 6;
- Fig. 8: eine perspektivische Darstellung der bei der Filtereinheit aus Fig. 1 verwendeten Deckelplatte;
- Fig. 9: einen vergrößerten Ausschnitt IX der Lagerung der Deckelplatte aus Fig. 2 im Wandbereich der Behälterwand des Behälterdeckels aus Fig. 1;
- Fig. 10: eine teilweise Draufsicht auf die Innenseite eines Wandabschnittes eines Behälterdeckels, bei welchem als Filtereinheit eine Pasteursche Schleife eingesetzt ist;
- Fig. 11: einen vertikalen Teilschnitt des Wandabschnittes aus Fig. 10 mit aufgesetzter Deckelplatte;
- Fig. 12: einen vergrößerten Ausschnitt aus Fig. 11 im Bereich des Ringsegmentes mit einem eingesetzten Rastelement;
- Fig. 13: eine perspektivische Ansicht einer mit Ringstegen versehenen Deckelplatte.

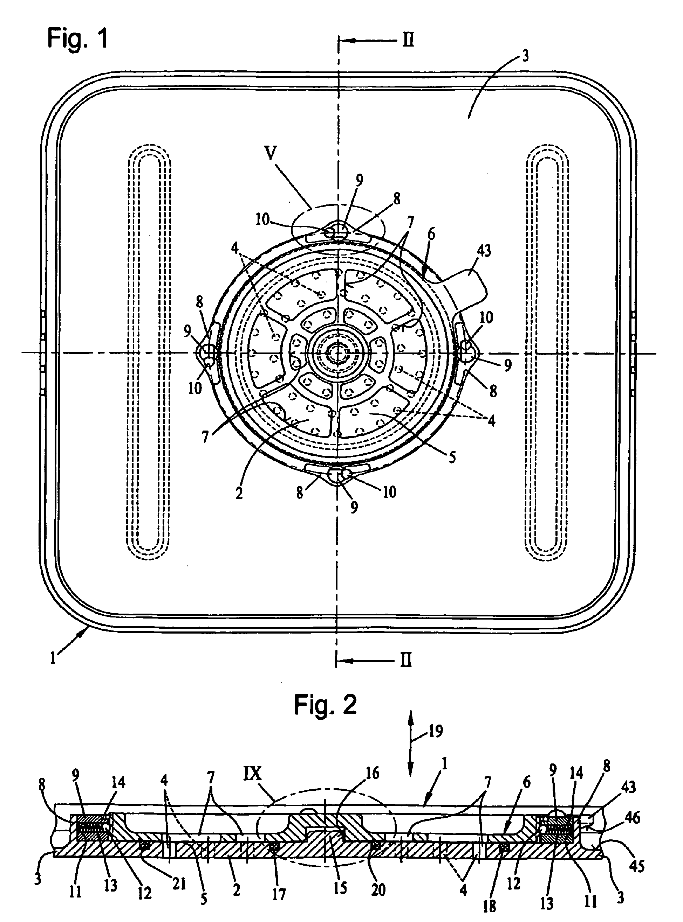

Fig. 1 zeigt eine Innenansicht eines Behälterdeckels 1, welcher dicht auf ein Gehäuseunterteil (in der Zeichnung nicht dargestellt) eines Sterilisierbehälters dicht aufsetzbar ist, wie dies aus dem Stand der Technik hinreichend bekannt ist. Der Behälterdeckel 1 weist bei der dargestellten Ausführungsvariante in einem zentralen Wandbereich 2 seiner Deckelwand 3 mehrere Belüftungsöffnungen 4 auf, welche innenseitig durch ein Filterblatt 5 abgedeckt sind. Da dieses Filterblatt 5 die Belüftungsöffnungen 4 verdeckt, sind die Belüftungsöffnungen 4 in Fig. 1 gestrichelt dargestellt.

Zur Fixierung des Filterblattes 5 ist bei der dargestellten Ausführungsvariante eine als Kreisscheibe ausgebildete Deckelplatte 6 vorgesehen, welche mehrere großflächige Durchbrüche 7 aufweist. Somit bildet bei der dargestellten Ausführungsvariante der Wandbereich 2 mit seinen Belüftungsöffnungen 4 zusammen mit dem Filterblatt 5 und der aufgesetzten Deckelplatte 6 eine Art Sterilbarriere, über welche ein innerer Sterilraum eines Sterilbehälters (in der Zeichnung nicht dargestellt) im Betrieb be-und entlüftet wird.

Des Weiteren ist aus Fig. 1 ersichtlich, dass im Umfangsbereich der Deckelplatte 6 mehrere Ringsegmente 8 vorgesehen sind, welche sich jeweils über einen Teilumfang der Deckelplatte 6 erstrecken. Jedes dieser Ringsegmente 8 ist mit einem Rastelement 9 versehen, welches beispielsweise jeweils mittels eines Spannstiftes 10 im jeweils zugehörigen Ringssegment 8 feststehend gehalten ist. Fig. 2 zeigt hierzu einen teilweisen Vertikalschnitt II-II aus Fig. 1 des Behälterdeckels 1 mit seinem Wandbereich 2 und der innenseitig aufgesetzten Deckelplatte 6.

Wie aus Fig. 2 ersichtlich ist, sind die Ringssegmente 8 radial außerhalb der Deckelplatte 6 in deren Umfangsbereich angeordnet. Jedes Ringssegment 8 weist eine Aufnahmebohrung 11 auf, in welche das jeweils zugehörige Rastelement 9 feststehend eingesetzt ist. Aus Fig. 2 ist dabei andeutungsweise erkennbar, dass jedes Rastelement 9 mit einer Rastkugel 12 sowie einer Axialdruckfeder 13 versehen ist, welche entsprechend in einer Radialbohrung 14 des jeweiligen Rastelementes 9 eingesetzt sind. Mittels dieser Rastkugel 12 ist das Deckelelement 6 innenseitig auf dem Wandbereich 2 bzw. der Deckelwand 3 des Behälterdeckels 1 rastend gehalten. Zur genauen Positionierung der Deckelplatte 6 zwischen den Ringsegmenten 8 bildet der Wandbereich 2 innenseitig einen zentralen Lagerzapfen 15, welcher entsprechend formschlüssig in eine als Sacklochbohrung ausgebildete Lagerbohrung 16 der Deckelplatte 6 eingreift.

Des Weiteren ist aus Fig. 2 andeutungsweise das Filterblatt 5 erkennbar. Zur Abdichtung dieses Filterblattes 5 sowohl radial nach innen zum Lagerzapfen 15 hin als auch radial nach außen zu den Ringsegmenten 8 hin sind jeweils O-Ringe 17 bzw. 18 vorgesehen, welche in dem in Fig. 2 dargestellten montierten Zustand der Deckelplatte 6 auf dem Wandbereich 2 unter axialer Vorspannung in Richtung des Doppelpfeils 19 dichtend unterseitig gegen das Filterblatt 5 drücken. Dementsprechend sind die beiden O-Ringe 17 und 18 jeweils in eine umlaufende Aufnahmenut 20 bzw. 21 des Wandbereiches 2 eingesetzt.

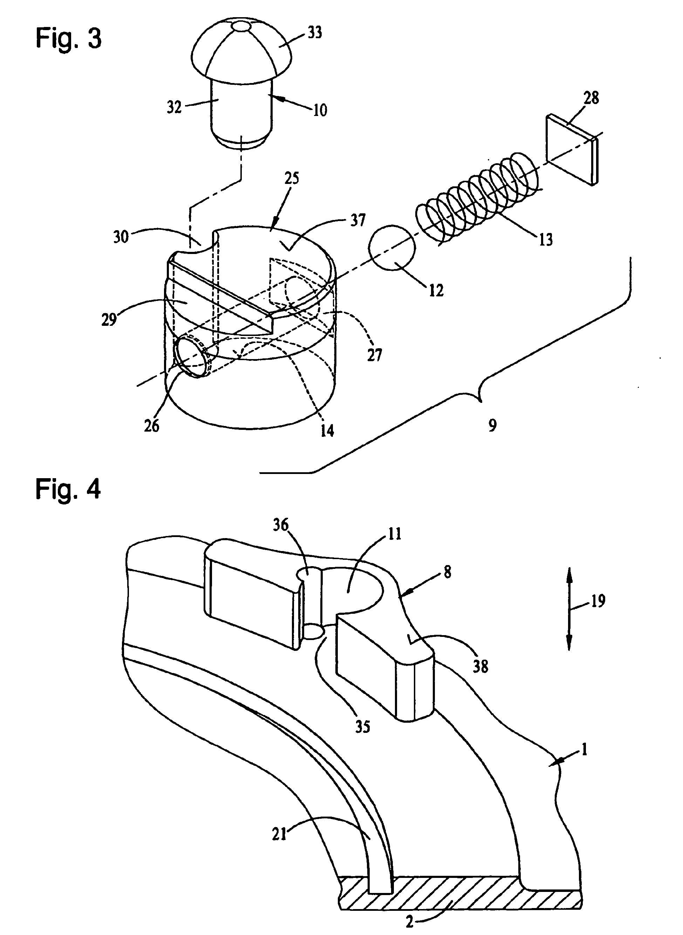

Beim vorliegenden Ausführungsbeispiel sind die aus Fig. 1 erkennbaren Ringssegmente 8 mit ihren Rastelementen 9 identisch ausgebildet, wobei die einzelnen Bestandteile der Rastelemente in Fig. 3 in einer perspektivischen Explosionsdarstellung beispielhaft dargestellt sind.

Das Rastelement 9 besteht bei der dargestellten Ausführungsvariante aus einem zentralen, im Wesentlichen zylindrischen Gehäuseblock 25, welcher zur Aufnahme der Rastkugel 12 sowie der Axialdruckfeder 13 die bereits zu Fig. 2 erwähnte Radialbohrung 14 aufweist. Diese Radialbohrung 14 ist etwa in der halben Höhe des Gehäuseblocks 25 angeordnet. Dabei bildet die Radialbohrung 14 in ihrem linken Endbereich einen radial nach innen vorstehenden Anschlagsteg 26, dessen Durchmesser kleiner ausgebildet ist als der Durchmesser der Rastkugel 12. Damit wird erreicht, dass die Rastkugel 12 in diesem Endbereich radial aus dem Gehäuseblock 25 um ein vorbestimmtes Maß herausragt, jedoch nicht aus der Radialbohrung 14 herausgedrückt werden kann.

Im diesem Anschlagsteg 26 axial gegenüber liegenden Endbereich der Radialbohrung 14 ist der Gehäuseblock 25 mit einer ebenen Ausfräsung 27 versehen, in welche eine Verschlussplatte 28 feststehend einsetzbar ist. Diese Verschlussplatte 28 kann dabei in der Ausfräsung 27 durch eine Pressverbindung oder auch durch eine Laserschweißverbindung fixiert sein. Durch diese Verschlussplatte 28 steht die Axialdruckfeder 13 im montierten Zustand unter axialer Vorspannung und stütz sich axial an der Verschlussplatte 28 ab, so dass die Rastkugel 12 mit einer vordefinierten Axialkraft gegen den Anschlagsteg 26 der Aufnahmebohrung 11 gedrückt wird.

Weiter ist aus Fig. 3 ersichtlich, dass der Gehäuseblock 25 oberseitig im Bereich des Anschlagsteges 26 eine weitere Ausfräsung 29 aufweist, in welche beispielsweise ein radial minimal vorstehender Bundsteg einer Deckelplatte hineinragen kann. In seinem rückwärtigen, seitlichen Bereich weist der Gehäuseblock 25 eine teilzylindrische Ausfräsung 30 auf, in welche der in Fig. 1 erkennbare Spannstift 10 einsetzbar ist. Dieser Spannstift 10 dient zur feststehenden Montage des Rastelementes 9 im in Fig. 4 perspektivisch dargestellten Ringsegment 8. Dementsprechend bildet der Spannstift 10 einen Spannzapfen 32 sowie ein Kopfteil 33.

Wie bereits oben erwähnt, zeigt Fig. 4 eine perspektivische Darstellung eines der Ringsegmente 8, welches oberseitig auf den Wandbereich 2 des Behälterdeckels 1 aufgesetzt bzw. an diesem angeformt ist. Weiter ist aus Fig. 4 noch die radial äußere Aufnahmenut 21 des Wandbereiches 2 erkennbar. Bei der dargestellten Ausführungsvariante des Behälterdeckels 1 ist dieser Wandbereich 2 podestartig erhöht ausgebildet. Dies ist allerdings nicht zwingend erforderlich. Weiter ist aus Fig. 4 ersichtlich, dass das Ringsegment 8 eine Art Aufnahmebohrung 11 aufweist, welche zur Aufnahmenut 21 hin einen sich über die komplette Höhe des Ringsegmentes 8 erstreckenden Durchbruch 35 bildet. In diese Aufnahmebohrung 11 ist das Rastelement 9 aus Fig. 3 mit seinem Gehäuseblock 25 passend einsetzbar. Zur Fixierung dieses Gehäuseblockes 25 in der Aufnahmebohrung 11 mittels des Spannstiftes 10 weist die Aufnahmebohrung 11 in ihrem Randbereich ebenfalls einen teilzylindrischen Bohrungsabschnitt 36 auf, welcher im montierten Zustand des Gehäuseblockes 25 in der Aufnahmebohrung 11 zusammen mit der Ausfräsung 30 des Gehäuseblocks 25 eine vollständig umlaufende zylindrische Bohrung bildet.

Im montierten Zustand wird somit der Gehäuseblock 25 durch den Spannstift 10 zum einen feststehend in der Aufnahmebohrung 11 gehalten und zum anderen wird durch die definierte Anordnung der Ausfräsung 30 am Umfang des Gehäuseblocks 25 und der definierten Anordnung des Bohrungsabschnittes 36 am Umfang der Aufnahmebohrung 11 eine radiale Ausrichtung der Radialbohrung 14 zur montierten Deckelplatte 6 erreicht, so dass auch die Rastkugel 12 im montierten Zustand radial nach innen weist, wie dies insbesondere aus den Fig. 1 und 2 erkennbar ist.

Bei der dargestellten Ausführungsvariante des Ringsegmentes 8 und des Rastelementes 9 sind diese in ihrer axialen Erstreckung des Doppelpfeils 19 gleich lang ausgebildet, so dass der Gehäuseblock 25 im montierten Zustand mit seiner Oberfläche 37 bündig mit der Oberfläche 38 des Ringsegmentes 8 abschließt. Durch die Ausbildung des Rastelementes 9 als separates Bauteil ist dieses in äußerst einfacher Weise herstellbar. Insbesondere ist es nicht notwendig, in das Ringsegment 8 eine Radialbohrung beispielsweise im Bereich der Aufnahmebohrung 11 vorzusehen, um dort die Rastkugel 12 zusammen mit der Axialdruckfeder 13 sowie der Verschlussplatte 28 einsetzen zu können. Dementsprechend kann dieses Ringsegment 8 auch einstückiger Bestandteil des Wandbereiches 2 bzw. des Behälterdeckels 1 sein.

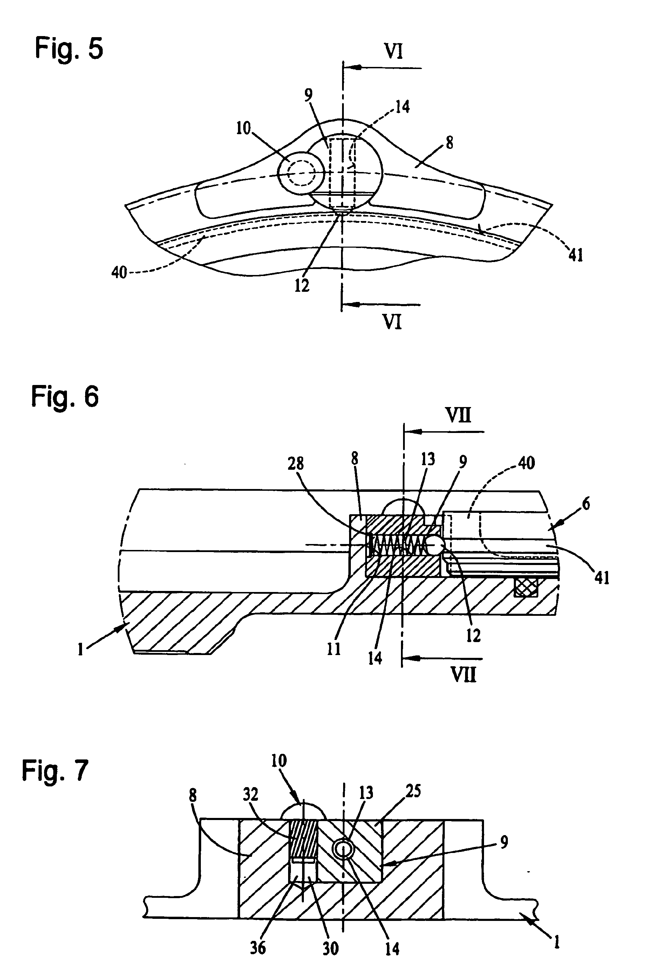

Weiter ist aus Fig. 1 hierzu ersichtlich, dass die Deckelplatte 6 mit äußerst geringem Spiel zwischen die bei dieser Ausführungsvariante vierfach vorgesehenen Ringsegmente 8 einsetzbar ist. Um die Deckelplatte 6 mit ausreichender Haltekraft auf dem Filterblatt 5 zu halten, weist die Deckelplatte 6 eine vertikal nach oben gerichtete, umlaufende Ringwand 40 auf (Fig. 6), welche mit einer umlaufenden Rastnut 41 versehen ist.

Hierzu zeigt Fig. 5 beispielhaft ein Ringsegment 8, in welches das Rastelement 9 feststehend eingesetzt ist. Dieses Rastelement 9 ist über den Spannstift 10 feststehend im Ringsegment 8 gehalten. Durch die entsprechende Umfangsanordnung sowohl der Ausfräsung 30 (Fig. 3) als auch des Bohrungsabschnittes 36 (Fig. 4) ist die Radialbohrung 14 des Rastelementes 9 radial ausgerichtet, so dass die Rastkugel 12 im montierten Zustand in die umlaufende Rastnut 41 eingreift. Dies ist insbesondere auch aus der Schnittdarstellung der Fig. 6 erkennbar. Die Radialbohrung 14 ist in diesem montierten Zustand des Rastelementes 9 in der Aufnahmebohrung 11 des Ringsegmentes 8 mittels der Verschlussplatte 28 verschlossen, so dass die Axialdruckfeder 13 die Rastkugel 12 in die Rastnut 41 der Ringwand 40 der Deckelplatte 6 drückt.

Aus der Schnittdarstellung der Fig. 7 ist erkennbar, dass die Ausfräsung 30 und der Bohrungsabschnitt 36 zusammen eine einheitliche Aufnahmebohrung für den Spannzapfen 32 des Spannstiftes 10 bilden. Der Durchmesser des Spannzapfens 32 kann dabei minimal größer ausgebildet sein als der Durchmesser der aus der Ausfräsung 30 und dem Bohrungsabschnitt 36 gebildeten Bohrung, so dass der Gehäuseblock 25 des Rastelementes 9 feststehend in der Aufnahmebohrung 11 des Ringsegmentes 8 gehalten ist. Weiter ist aus Fig. 7 noch die Axialdruckfeder 13 in der Radialbohrung 14 des Gehäuseblockes 25 erkennbar.

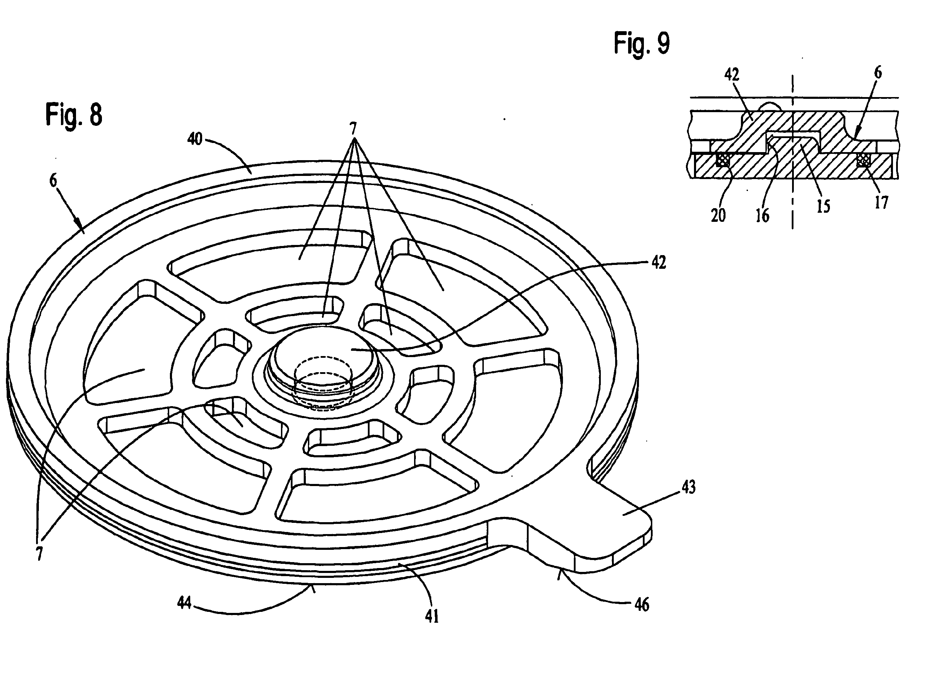

Fig. 8 zeigt eine perspektivische Darstellung der Druckplatte 6 aus Fig. 1. Aus Fig. 8 ist die umlaufende Ringwand 40 mit der zumindest teilweise umlaufenden Rastnut 41 erkennbar. Weiter sind in Fig. 8 auch die großflächigen Durchbrüche 7 dargestellt. Im Zentrum bildet die Deckelplatte 6 eine zapfenartige Erhöhung 42, in deren Bereich unterseitig die bereits zu Fig. 2 erwähnte Lagerbohrung 16 (Fig. 9) vorgesehen ist.

Weiter ist aus Fig. 8 erkennbar, dass die Deckelplatte 6 beim dargestellten Ausführungsbeispiel an ihrem umlaufenden Ringsteg 40 mit einem radial nach außen vorstehenden, plattenartig ausgebildeten Griffteil 43 versehen ist. Dieses Griffteil 43 weist zur unteren Auflagefläche 44 der Deckelplatte 6 hin einen vertikalen Abstand auf, so dass im montierten Zustand der Deckelplatte 6 auf dem Wandabschnitt 2 das Griffteil 43 von der Oberfläche 45 der Deckelwand 3 einen Abstand aufweist, wie dies andeutungsweise aus Fig. 2 erkennbar ist. Um das Griffteil 43 mit den Fingern leichter hintergreifen zu können, weist das Griffteil 43 zu seinem radial äußeren Ende hin eine "untere" Abschrägung 46 auf, wodurch der Abstand zur Oberfläche 45 der Deckelwand 3 vergrößert ist. Mittels dieses Griffteils 43 lässt sich die Deckelplatte 6 aus ihrem in Fig. 2 dargestellten, eingerasteten Zustand wesentlich einfacher aus der Rastverbindung zwischen den Rastkugeln 12 und der Rastnut 41 lösen.

Fig. 9 zeigt des Weiteren in vergrößerter Darstellung den Lagerbereich der Deckelplatte 6 auf dem Wandabschnitt 2. Es ist einerseits die vertikal nach oben vorstehende Erhöhung 42 erkennbar, in deren Bereich die Lagerbohrung 16 der Deckelplatte 6 angeordnet ist. In diese Lagerbohrung 16 greift der Lagerzapfen 15 des Wandbereiches 2 passend ein. Weiter ist aus Fig. 9 noch der innere O-Ring 17 erkennbar, welcher entsprechend in die Aufnahmenut 20 eingesetzt ist. Durch den Lagerzapfen 15 und die Lagerbohrung 16 ist die Deckelplatte 6 relativ positionsgenau auf dem Wandbereich 2 bzw. im Behälterdeckel 1 positionierbar. Entsprechend wird auch die Deckelplatte 6 über die Rastelemente 9 gleichmäßig feststehend am Umfang auf dem Behälterdeckel 1 bzw. im Behälterdeckel 1 gehalten.

Anstatt eines solchen Griffteils 43 zum Abnehmen der Deckelplatte 6 vom Wandbereich 2 des Behälterdeckels 1 oder ergänzend hierzu kann die umlaufende Nut 41 auch unterbrochen ausgebildet sein.

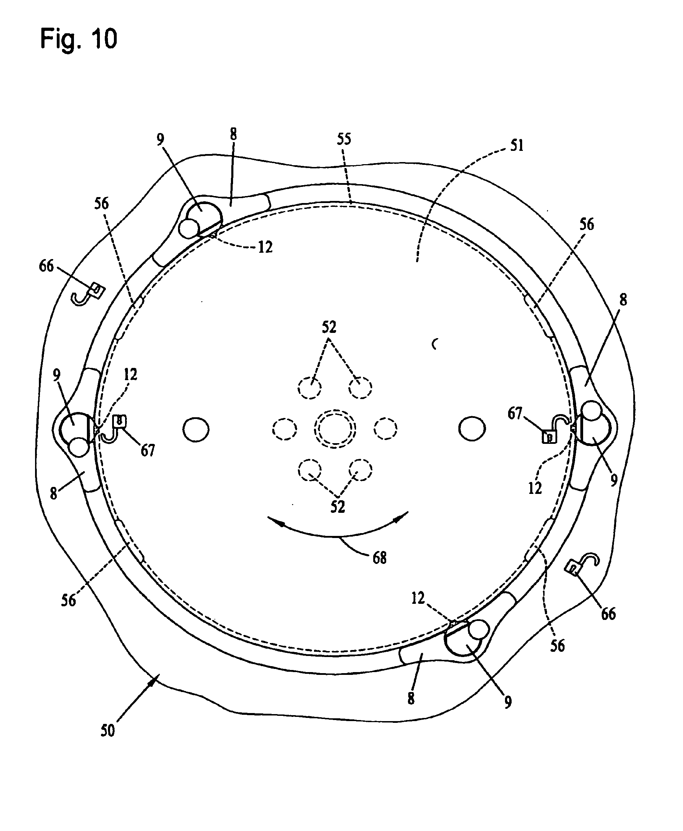

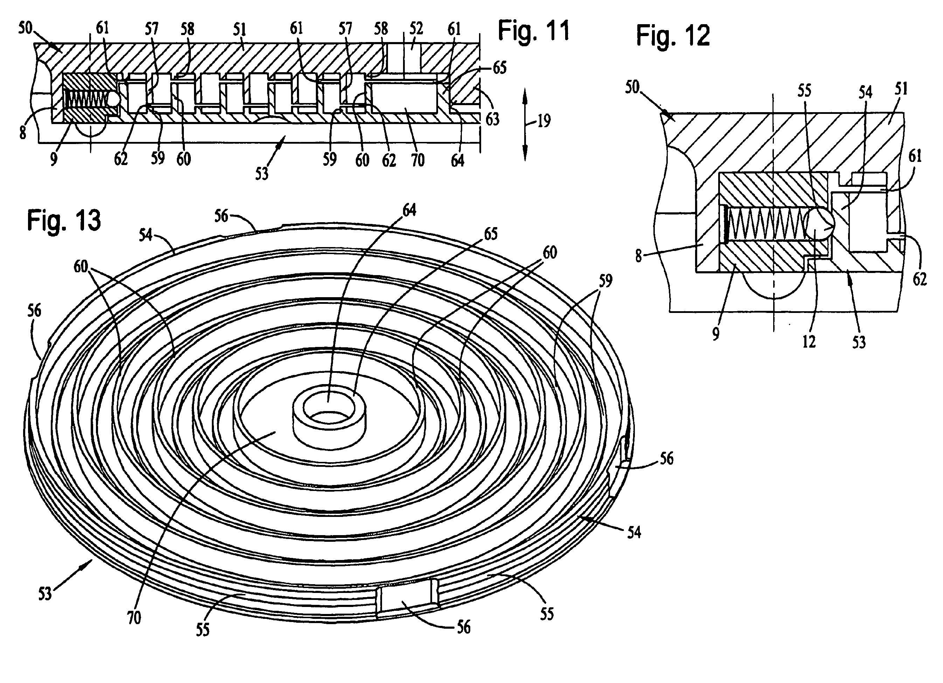

Hierzu zeigt Fig. 10 eine Draufsicht einer weiteren Variante eines Behälterdeckels 50 in Innenansicht, welcher zentral einen Wandabschnitt 51 bildet, der mit inneren Belüftungsöffnungen 52 versehen ist. Diese Belüftungsöffnungen 52 sind auch bei der Ausführungsvariante nach Fig. 10 mittels einer Deckelplatte 53 abgedeckt, welche allerdings zusammen mit entsprechend ausgebildeten Teilen des Wandabschnittes 51 eine sog. Pasteursche Schleife bildet, deren grundsätzliche Ausgestaltung aus dem Stand der Technik bekannt und in Fig. 12 im Teilschnitt dargestellt ist.

Wie aus Fig. 10 ersichtlich ist, weist die Deckelplatte 53 kein nach außen vorstehendes Griffteil auf. Diese Deckelplatte 53 bildet eine umlaufenden äußere Ringwand 54 (Fig. 12), welche mit einer umlaufenden Rastnut 55 (Fig. 10) versehen ist. Diese Rastnut 55, welche in Fig. 10 in gestrichelten Linien erkennbar ist, ist durch insgesamt vier Aussparungen 56 unterbrochen, deren radiale Tiefe größer ist als die radiale Tiefe der Rastnut 55.

Es ist aus Fig. 10 erkennbar, dass bei dieser Ausführungsvariante der Behälterdeckels 50 innenseitig ebenfalls mit vier identisch ausgebildeten Ringsegmenten 8 versehen ist, in welche ebenfalls jeweils ein Rastelement 9 eingesetzt ist. Bei dieser dargestellten Ausführungsvariante sind die Ringsegmente 8 mit ihren Rastelementen 9 paarweise in unterschiedlichen Winkelabständen zueinander im Umfangsbereich der Deckelplatte 53 angeordnet. Entsprechend dieser Abstände sind auch die Aussparungen 56 der Rastnut 55 in der umlaufenden Ringwand 54 angeordnet.

In der in Fig. 10 dargestellten Position bzw. Winkelstellung ist die Deckelplatte 53 mit ihrer umlaufenden Rastnut 55 in den andeutungsweise erkennbaren Rastkugeln 12 der Rastelemente 9 feststehend eingerastet. Diesen rastenden Eingriff zeigen insbesondere die beiden Teilschnitte der Fig. 11 und 12. So ist aus der Schnittdarstellung der Fig. 11 eines der Ringsegmente 8 zusammen mit einem aufgenommenen Rastelement 9 erkennbar. Fig. 12 zeigt diesen Teil aus Fig. 11 in vergrößerter Darstellung. Die Rastkugel 12 greift in diesem montierten Zustand in die umlaufende Rastnut 55 des Randsteges 54 formschlüssig ein. Die Deckelplatte 53 wird durch diesen formschlüssigen Eingriff in der in Fig. 11 und 12 dargestellten axialen Position gegenüber dem Wandabschnitt 51 des Behälterdeckels 50 gehalten.

Wie insbesondere aus Fig. 11 ersichtlich ist, weist dieser Wandabschnitt 51 des Behälterdeckels 50 im Bereich der Deckelplatte 53 entsprechende Ringstege 57 und 58 auf, welche in ihrer axialen Erstreckung des Doppelpfeils 19 unterschiedlich hoch ausgebildet sind. Entsprechend dieser Höhenausbildung weist die Deckelplatte 53 zum Behälterdeckel 50 hin ebenfalls entsprechende Ringstege 59 und 60 auf, welche in ihren Durchmessern und in ihrer Höhe den Ringstegen 57 und 58 entsprechend deckungsgleich angepasst sind. Aufgrund der rastenden axialen Fixierung der Deckelplatte 53 durch die Rastkugeln 12 wird die Deckelplatte 53 zum Behälterdeckel 50 bzw. dessen Wandabschnitt 51 in einem Abstand gehalten, so dass sich zwischen den Ringstegen 57 und 59 bzw. 58 und 60 radiale Spalte 61 bzw. 62 ergeben.

Im radial inneren Bereich mündet der innerste Radialspalt 61 in eine Ringkammer 70, welche wiederum mit den Belüftungsöffnungen 52 in Verbindung steht, wie dies für eine der Belüftungsöffnungen 52 aus Fig. 11 ersichtlich ist. So besteht über diese Belüftungsöffnungen 52 und die radialen Ringspalten 61 und 62 eine labyrinthartige Verbindung zu einem geschlossenen Sterilisierraum eines Sterilisierbehälters, wie dies aus dem Stand der Technik gemeinhin bekannt ist. Weiter ist aus Fig. 11 noch ersichtlich, dass der Wandabschnitt 51 des Behälterdeckels 50 einen zentralen Lagerzapfen 63 bildet, welcher in eine entsprechend zugehörige Lagerbohrung 64 der Deckelplatte 53 passend eingreift. Somit wird auch die Deckelplatte 53 über diesen Lagerzapfen 63 und die Lagerbohrung 64 relativ positionsgenau innenseitig am Behälterdeckel 50 bzw. dessen Wandabschnitt 51 gehalten.

Fig. 13 zeigt der Vollständigkeit halber nochmals eine perspektivische "Draufsicht" der Deckelplatte 53. Einerseits sind in dieser Darstellung die vertikal erhöhten Ringstege 60 sowie die in ihrer Höhe kleiner ausgebildeten Ringstege 59 erkennbar, welche abwechselnd in radialer Richtung von außen nach innen aufeinander folgen. Auch ist der innere Ringsteg 65 erkennbar, welcher einerseits die zentrale Lagerbohrung 64 und andererseits einen Teil der Ringkammer 70 definiert.

Weiter sind aus Fig. 13 die bereits zu Fig. 10 erwähnten Aussparungen 56 erkennbar. Die Aussparungen sind in ihrer radialen Tiefe größer ausgebildet als die umlaufende Ringnut 55 der äußeren Ringwand 54 der Deckelplatte 53. Dementsprechend werden die Rastkugeln 12 bei entsprechender Ausrichtung dieser Aussparungen 56 auf die Rastkugeln 12 freigegeben, so dass die Deckelplatte 53 ohne größere Mühe vom Wandabschnitt 51 des Behälterdeckels 50 abgehoben werden kann.

Zur Erleichterung der Ausrichtung dieser Aussparungen 56 auf die entsprechend zugehörigen Rastkugeln 12 bzw. Rastelemente 9, können sowohl im Umgebungsbereich der Deckelplatte 53 innenseitig auf dem Behälterdeckel 50 als auch auf der Deckelplatte 53 entsprechende Markierungen - beispielsweise in Form von geöffneten Bügelschlössern 66 bzw. 67 - vorgesehen sein. Durch entsprechendes Drehen der Deckelplatte 53 in eine der Richtungen des Doppelpfeils 68 (Fig. 10) sind somit die Aussparungen 56 in Umfangsrichtung mit den Rastkugeln 12 in Überdeckung bringbar. Dabei ist die korrekte Winkelposition der Deckelplatte 53 dann erreicht, wenn die Markierungen 67 der Deckelplatte 53 in radialer Richtung mit den Markierungen 66 des Behälterdeckels 50 fluchten.

Insoweit ist auch eine Montage in einer korrekten rastenden Winkelstellung der Deckelplatte 53 relativ zu den umfänglich angeordneten Ringsegmenten 8 mit ihren Rastelementen 9 einstellbar, indem die Markierungen 67 und 66 beim Aufsetzen beispielsweise in die in Fig. 10 dargestellte Position gebracht werden. Dabei können diese vorzugsweise in einer Winkelstellung auf der Deckelplatte 53 angeordnet sein, in welcher sie beispielsweise in radialer Richtung mit zwei der Ringsegmente 8 mit ihren Rastelementen 9 fluchten, wie dies in Fig. 10 beispielhaft dargestellt ist. Dies wird dadurch ermöglicht, dass die Ringssegmente 8 mit ihren Rastelementen 9 paarweise in vorbestimmten, unterschiedlichen Umfangsabständen zueinander angeordnet sind.

## Patentansprüche

1. Sterilisierbehälter bestehend aus einem kastenartigen Unterteil und einem abnehmbaren Behälterdeckel (1, 50), welche einen geschlossenen, luftdichten Sterilraum bilden, wobei in einem Wandbereich (2, 51) des Sterilisierbehälter Belüftungsöffnungen (4, 52) vorgesehen sind, durch welche im geschlossenen Zustand ein Luftaustausch zwischen dem Sterilraum und der Umgebung stattfindet, und wobei der Wandbereich (2, 51) im Bereich der Belüftungsöffnungen (4, 52) zusammen mit einer Deckelplatte (6, 53) Bestandteil einer Sterilbarriere ist, welche eine Sterilisation der durch die Belüftungsöffnungen (4, 52) in den Sterilraum gelangenden Luft bewirkt, wobei die Deckelplatte (6, 53) durch wenigstens eine federnde Schnapp- oder Rastverbindung (9, 41, 55) lösbar mit dem Wandbereich in Eingriff steht,
**dadurch gekennzeichnet,**
**dass** die Deckelplatte (6, 53) als runde Scheibe mit einer umlaufenden Ringwand (40, 54) ausgebildet ist, die außenseitig eine umlaufende Rastnut (41, 55) aufweist, in welche mehrere im Umfangsbereich der Deckelplatte (6, 53) angeordnete Rastelemente (9) lösbar eingreifen und,
**dass** die Rastelemente (9) als jeweils separate Baueinheit in rechtwinklig zur Deckelplatte (6, 53) verlaufende Aufnahmebohrungen (34) von axial über den Wandbereich (2, 51) vorstehenden Ringssegmenten (8) feststehend angeordnet sind.

2. Sterilisierbehälter nach Anspruch 1, **dadurch gekennzeichnet, dass** die Rastelemente (9) jeweils aus einem etwa zylindrischen Gehäuseblock (25) bestehen, welcher mit einer quer verlaufenden Radialbohrung (14) versehen ist, in welcher eine radial aus dem Gehäuseblock (25) teilweise vorstehende und im montierten Zustand in die Rastnut (41, 55) eingreifende Rastkugel (12) angeordnete ist und,
dass die Rastkugel (12) durch eine in der Radialbohrung (14) angeordnete Axialdruckfeder (13) in ihrer Eingriffsposition federelastisch nachgiebig gehalten ist.

3. Sterilisierbehälter nach Anspruch 2, **dadurch gekennzeichnet, dass** die Radialbohrung (14) im Bereich der teilweise radial vorstehenden Rastkugel (12) einen nach innen gerichteten Anschlagsteg (26) aufweist, durch welchen der maximale radiale Vorstand der Rastkugel (12) bestimmt ist.

4. Sterilisierbehälter nach Anspruch 2 oder 3, **dadurch gekennzeichnet, dass** die Radialbohrung (14) des Gehäuseblocks (25) in ihrem der Rastkugel (12) gegenüber liegenden Endbereich mit einer Verschlussplatte (28) versehen ist, welche in einer Ausfräsung (27) des Gehäuseblocks (25) feststehend aufgenommen ist und an welcher sich die Axialdruckfeder (13) axial abstützt.

5. Sterilisierbehälter nach einem der Ansprüche 1 bis 4, **dadurch gekennzeichnet, dass** zwischen der Deckelplatte (6) und dem Wandbereich (2) des Behälterdeckels (1) ein die Belüftungsöffnungen (4) innenseitig abdeckendes Filterblatt (5) vorgesehen ist und,
dass die Rastverbindung (9, 41) zwischen der Deckelplatte (6) und dem Wandbereich (2) eine Klemmkraft bewirkt, mit welcher das Filterblatt (5) gegen den Wandbereich (2) des Behälterdeckels (1) gedrückt wird.

6. Sterilisierbehälter nach einem der Ansprüche 1 bis 5, **dadurch gekennzeichnet, dass** die Deckelplatte (6) ein radial vorstehendes Griffteil (43) aufweist, welches im montierten Zustand zwischen zwei benachbarten Ringsegmenten (8) der Deckelplatte (6) angeordnet ist und,
dass das Griffteil (43) zur inneren Oberfläche (45) der Deckelwand (3) des Behälterdeckels (1) einen Abstand aufweist.

7. Sterilisierbehälter nach Anspruch 6, **dadurch gekennzeichnet, dass** das Griffteil (42) auf seiner zur Oberfläche (45) des Behälterdeckels (6) hin liegenden "Unterseite" eine Abschrägung (46) aufweist, in dessen radial äußerem Endbereich der Abstand zur Oberfläche (45) des Behälterbodens (6) größer ist als in dessen radial innerem Bereich.

8. Sterilisierbehälter nach einem der Ansprüche 1 bis 4, **dadurch gekennzeichnet, dass** die Sterilbarriere aus einer Pasteurschen Schleife gebildet ist, welche aus in radialer Richtung wechselseitig axial (Pfeil 19) ineinander greifende Ringstegen (57, 58 bzw. 59, 60) des Wandbereichs (51) einerseits und der Deckelplatte (53) andererseits gebildet wird und,
dass die Rastnut (55) durch mehrere Aussparungen (56) unterbrochen ist, deren Anzahl und Anordnung der Anzahl und der Anordnung der Rastelemente (9) entsprechen und, dass die Aussparungen (56) ausgehend von der Rastnut (55) zum Wandbereich (51) des Behälterdeckels (50) hin offen sind.

9. Sterilisierbehälter nach Anspruch 8, **dadurch gekennzeichnet, dass** die Deckelplatte (53) relativ zum Wandbereich (51) des Behälterdeckels (50) drehbar ist (Doppelpfeil 68) und, dass die Aussparungen (56) durch entsprechende Einstellung der relativen Winkelstellung der Deckelplatte (53) zum Wandbereich (51) mit den im Umfangsbereich der Deckelplatte (53) angeordneten Rastelementen (9) in Überdeckung und mit der Rastnut (55) außer Eingriff bringbar sind.

10. Sterilisierbehälter nach Anspruch 9, **dadurch gekennzeichnet, dass** im Umgebungsbereich der Deckelplatte (53) auf dem Behälterdeckel (50) und im Randbereich der Deckelplatte (53) Markierungen (66 bzw. 67) vorgesehen sind, welche radial fluchtend zueinander angeordnete sind, sofern sich die Rastelemente im Umfangsbereich der jeweils zugeordneten Aussparung (56) befinden.

11. Sterilisierbehälter nach einem der Ansprüche 1 bis 10, **dadurch gekennzeichnet, dass** der Wandbereich (2, 51) einen zentralen, zur Deckelplatte (6, 53) hin vorstehenden Lagerzapfen (15, 63) aufweist, auf welchem die Deckelplatte (6, 53) im montierten Zustand mit einer Lagerbohrung (16, 64) konzentrisch zu den am Unfang angeordneten Ringsegmenten (8) im Wandbereich (2, 51) aufgenommen ist.

12. Sterilisierbehälter nach einem der Ansprüche 1 bis 11, **dadurch gekennzeichnet, dass** der Abstand benachbarter Rastelemente (9) jeweils derart gewählt ist, dass dazwischen ein manuelles Ergreifen der Deckelplatte (6, 53) an deren Umfang ermöglicht wird.

## Claims

1. Sterilization container composed of a box-like lower part and of a removable container cover (1, 50) that form a closed, airtight sterile space, wherein a wall area (2, 51) of the sterilization container is provided with ventilation openings (4, 52) through which, in the closed state, an exchange of air takes place between the sterile space and the environment, and wherein the wall area (2, 51) in the area of the ventilation openings (4, 52) is, together with a cover plate (6, 53), a component part of a sterile barrier that effects a sterilization of the air passing through the ventilation openings (4, 52) into the sterile space, wherein the cover plate (6, 53) engages releasably with the wall area by means of at least one resilient snap-fit or locking connection (9, 41, 55), **characterized in that** the cover plate (6, 53) is designed as a round disc with a peripheral annular wall (40, 54) which, on the outside, has a peripheral locking groove (41, 55) in which several locking elements (9) arranged in the peripheral area of the cover plate (6, 53) releasably engage, and **in that** the locking elements (9), each as a separate structural unit, are arranged in a fixed position in receiving bores (34) of ring segments (8) protruding axially above the wall area (2, 51), said receiving bores (34) extending at right angles to the cover plate (6, 53).

2. Sterilization container according to Claim 1, **characterized in that** the locking elements (9) are each composed of an approximately cylindrical housing block (25), which is provided with a transversely extending radial bore (14) in which a locking ball (12) is arranged that partially protrudes radially from the housing block (25) and that engages in the locking groove (41, 55) in the mounted state, and **in that** the locking ball (12) is held elastically and resiliently in its engagement position by an axial compression spring (13) arranged in the radial bore (14).

3. Sterilization container according to Claim 2, **characterized in that** the radial bore (14), in the area of the partially radially protruding locking ball (12), has an inwardly directed stop web (26), by which the maximum radial protrusion of the locking ball (12) is defined.

4. Sterilization container according to Claim 2 or 3, **characterized in that** the radial bore (14) of the housing block (25) is provided, in its end area lying opposite the locking ball (12), with a closure plate (28) which is received in a fixed position in a countersink (27) of the housing block (25) and on which the axial compression spring (13) is axially supported.

5. Sterilization container according to one of Claims 1 to 4, **characterized in that** a filter sheet (5) covering the ventilation openings (4) on the inside is provided between the cover plate (6) and the wall area (2) of the container cover (1), and **in that** the locking connection (9, 41) between the cover plate (6) and the wall area (2) brings about a clamping force, with which the filter sheet (5) is pressed against the wall area (2) of the container cover (1).

6. Sterilization container according to one of Claims 1 to 5, **characterized in that** the cover plate (6) has a radially protruding grip part (43) which, in the mounted state, is arranged between two adjacent ring segments (8) of the cover plate (6), and **in that** the grip part (43) is at a distance from the inner surface (45) of the cover wall (3) of the container cover (1).

7. Sterilization container according to Claim 6, **characterized in that** the grip part (42), on its "underside" located towards the surface (45) of the container cover (6), has a bevel (46) which, in its radially outer end area, is at a distance from the surface (45) of the container bottom (6) that is greater than the distance in its radially inner area.

8. Sterilization container according to one of Claims 1 to 4, **characterized in that** the sterile barrier is formed by a Pasteur loop, which is formed, on the one hand, by annular webs (57, 58 and 59, 60) of the wall area (51) that engage in one another axially (arrow 19) alternatingly in a radial direction, and, on the other hand, by the cover plate (53), and **in that** the locking groove (55) is interrupted by a plurality of recesses (56), of which the number and arrangement correspond to the number and arrangement of the locking elements (9), and **in that** the recesses (56) are open, starting from the locking groove (55), towards the wall area (51) of the container cover (50).

9. Sterilization container according to Claim 8, **characterized in that** the cover plate (53) is rotatable (double arrow 68) relative to the wall area (51) of the container cover (50), and **in that** the recesses (56) can be caused to overlap the locking elements (9) arranged in the peripheral area of the cover plate (53) and can be disengaged from the locking groove (55) by corresponding adjustment of the relative angular position of the cover plate (53) in relation to the wall area (51).

10. Sterilization container according to Claim 9, **characterized in that**, in the peripheral area of the cover plate (53), markings (66 and 67) are provided on the container cover (50) and in the edge area of the cover plate (53), said markings being arranged radially aligned with one another as long as the locking elements are located in the peripheral area of the respectively associated recess (56).

11. Sterilization container according to one of Claims 1 to 10, **characterized in that** the wall area (2, 51) has a central bearing pin (15, 63), which protrudes towards the cover plate (6, 53) and on which the cover plate (6, 53), in the mounted state, is received with a bearing bore (16, 64) concentric with respect to the ring segments (8) in the wall area (2, 51), which ring segments are arranged on the circumference.

12. Sterilization container according to one of Claims 1 to 11, **characterized in that** the distance between adjacent locking elements (9) is in each case chosen such that the cover plate (6, 53) can be grasped manually between them at the circumference of said cover plate.

## Revendications

1. Récipient de stérilisation constitué d'une partie inférieure en forme de caisse et d'un couvercle de récipient amovible (1, 50), lesquels forment un espace stérile fermé étanche à l'air, des ouvertures de ventilation (4, 52) étant prévues dans une région de paroi (2, 51) du récipient de stérilisation, à travers lesquelles, dans l'état fermé, un échange d'air a lieu entre l'espace stérile et l'environnement, la région de paroi (2, 51), dans la région des ouvertures de ventilation (4, 52), conjointement avec une plaque de couvercle (6, 53), faisant partie d'une barrière stérile qui produit une stérilisation de l'air parvenant dans l'espace stérile à travers les ouvertures de ventilation (4, 52), la plaque de couvercle (6, 53) étant en prise de manière amovible avec la région de paroi par au moins une connexion élastique par encliquetage ou par emboîtement (9, 41, 55),
**caractérisé en ce que**
la plaque de couvercle (6, 53) est réalisée sous forme de disque rond avec une paroi annulaire périphérique (40, 54), qui présente du côté extérieur une rainure d'emboîtement périphérique (41, 55), dans laquelle s'engagent de manière amovible plusieurs éléments d'emboîtement (9) disposés dans la région périphérique de la plaque de couvercle (6, 53), et
les éléments d'emboîtement (9) sont disposés de manière fixe sous forme d'unité structurelle séparée respective dans des alésages de réception (34), s'étendant à angle droit par rapport à la plaque de couvercle (6, 53), de segments annulaires (8) faisant saillie axialement au-delà de la région de paroi (2, 51).

2. Récipient de stérilisation selon la revendication 1, **caractérisé en ce que** les éléments d'emboîtement (9) se composent à chaque fois d'un bloc boîtier approximativement cylindrique (25) qui est muni d'un alésage radial s'étendant transversalement (14) dans lequel est disposée une bille d'emboîtement (12) faisant saillie en partie radialement hors du bloc boîtier (25) et s'engageant dans l'état monté dans la rainure d'emboîtement (41, 55), et
la bille d'emboîtement (12) est retenue de manière souple et élastiquement flexible dans sa position d'engagement par un ressort de compression axial (13) disposé dans l'alésage radial (14).

3. Récipient de stérilisation selon la revendication 2, **caractérisé en ce que** l'alésage radial (14) présente, dans la région de la bille d'emboîtement (12) saillant en partie radialement, une nervure de butée (26) orientée vers l'intérieur, qui détermine le dépassement maximum radial de la bille d'emboîtement (12).

4. Récipient de stérilisation selon la revendication 2 ou 3, **caractérisé en ce que** l'alésage radial (14) du bloc boîtier (25) est muni, dans sa région d'extrémité située à l'opposé de la bille d'emboîtement (12), d'une plaque de fermeture (28) qui est reçue de manière fixe dans un fraisage (27) du bloc boîtier (25) et contre laquelle s'appuie axialement le ressort de compression axial (13).

5. Récipient de stérilisation selon l'une quelconque des revendications 1 à 4, **caractérisé en ce qu'**entre la plaque de couvercle (6) et la région de paroi (2) du couvercle de récipient (1) est prévue une lame de filtre (5) recouvrant les ouvertures de ventilation (4) du côté intérieur et la connexion par emboîtement (9, 41) entre la plaque de couvercle (6) et la région de paroi (2) produit une force de serrage avec laquelle la lame de filtre (5) est pressée contre la région de paroi (2) du couvercle de récipient (1).

6. Récipient de stérilisation selon l'une quelconque des revendications 1 à 5, **caractérisé en ce que** la plaque de couvercle (6) présente une partie de préhension saillant radialement (43) qui, dans l'état monté, est disposée entre deux segments annulaires adjacents (8) de la plaque de couvercle (6), et
la partie de préhension (43) présente une distance à la surface intérieure (45) de la paroi de couvercle (3) du couvercle de récipient (1).

7. Récipient de stérilisation selon la revendication 6, **caractérisé en ce que** la partie de préhension (42) présente, sur son "côté inférieur" tourné vers la surface (45) du couvercle de récipient (6), un biseautage (46), dans la région d'extrémité radialement extérieure duquel la distance à la surface (45) du fond du récipient (6) est plus grande que dans sa région radialement interne.

8. Récipient de stérilisation selon l'une quelconque des revendications 1 à 4, **caractérisé en ce que** la barrière stérile est formée d'une boucle de Pasteur qui est formée de nervures annulaires (57, 58, respectivement 59, 60) de la région de paroi (51), venant en prise les unes dans les autres axialement en alternance dans la direction radiale (flèche 19) d'une part et de la plaque de couvercle (53) d'autre part, et
**en ce que** la rainure d'emboîtement (55) est interrompue par plusieurs évidements (56) dont le nombre et l'agencement correspondent au nombre et à l'agencement des éléments d'emboîtement (9) et **en ce que** les évidements (56) sont ouverts à partir de la rainure d'emboîtement (55) vers la région de paroi (51) du couvercle de récipient (50).

9. Récipient de stérilisation selon la revendication 8, **caractérisé en ce que** la plaque de couvercle (53) peut tourner par rapport à la région de paroi (51) du couvercle de récipient (50) (double flèche 68) et **en ce que** les évidements (56) par un ajustement correspondant de la position angulaire relative de la plaque de couvercle (53) par rapport à la région de paroi (51) peuvent être amenés en coïncidence avec les éléments d'emboîtement (9) disposés dans la région périphérique de la plaque de couvercle (53) et hors d'engagement avec la rainure d'emboîtement (55).

10. Récipient de stérilisation selon la revendication 9, **caractérisé en ce que** dans la région périphérique de la plaque de couvercle (53) sur le couvercle de récipient (50) et dans la région de bord de la plaque de couvercle (53) sont prévus des marquages (66 respectivement 67) qui sont disposés de manière alignée radialement les uns avec les autres lorsque les éléments d'emboîtement se trouvent dans la région périphérique de l'évidement respectivement associé (56).

11. Récipient de stérilisation selon l'une quelconque des revendications 1 à 10, **caractérisé en ce que** la région de paroi (2, 51) présente un tourillon de palier central (15, 63) faisant saillie vers la plaque de couvercle (6, 53) sur lequel la plaque de couvercle (6, 53) est reçue dans l'état monté avec un alésage de palier (16, 64) concentriquement aux segments annulaires (8) disposés à la périphérie dans la région de paroi (2, 51).

12. Récipient de stérilisation selon l'une quelconque des revendications 1 à 11, **caractérisé en ce que** la distance entre des éléments d'emboîtement adjacents (9) est choisie à chaque fois de telle sorte qu'il soit possible de saisir manuellement entre eux la plaque de couvercle (6, 53) au niveau de sa périphérie.
